# EUROPEAN PATENT SPECIFICATION

(11) **EP 0 443 090 B1**
(45) Date of publication and mention of the grant of the patent: **10.01.1996**
(21) Application number: 90118952.2
(22) Date of filing: 04.10.1990
(51) Int. Cl.: A61K 35/78

(54) **Agent for preventing infection with methicillin-resistant staphylococcus**
Mittel zur Verhütung von Infektionen mit Methicillin-beständigem Staphylococcus
Agents pour la prévention d'infections des staphylocoques résistant aux methicillin

(30) Priority: 23.02.1990 JP 42984/90
(43) Date of publication of application: 28.08.1991
(73) Proprietor: MITSUI NORIN CO., LTD., Tokyo (JP); Shimamura, Tadakatsu, Tokyo (JP)
(72) Inventor: Shimamura, Tadakatsu, Shibuya-ku Tokyo (JP)
(74) Representative: Türk, Gille, Hrabal, Leifert

(56) References cited:
- EP-A- 0 397 914
- BIOLOGICAL ABSTRACTS, vol. 73, no. 1, 1982, page 248, abstract no. 2351, Philadelphia, PA, US; E. RYU: "Prophylactic effect of tea on pathogenic microorganism infection to humans and animals: 1. Growth inhibitive and bactericidal effect of tea on food poisoning and other pathogenic enterobacteria in vitro"
- BIOLOGICAL ABSTRACTS, vol. 76, no. 4, 1983, page 2633, abstract no. 24362, Philadelphia, PA, US; E. RYU et al.: "Inhibition of growth of selected bacteria by incorporating powdered tea in the medium"
- BIOLOGICAL ABSTRACTS, vol. 88, no. 2, 1989, page AB-328, abstract no. 129649, Philadelphia, PA, US; M. TODA et al.: "Antibacterial and bactericidalactivities of Japanese green tea"
- CHEMICAL ABSTRACTS, vol. 113, no. 3, 16th July 1990, page 525, abstract no.22430u, Columbus, Ohio, US; Y. HARA et al.: "Studies on antibacterial effects of tea polyphenols. III. Antibacterial activities of tea polyphenols against foodborne pathogenic bacteria"
- "HAGERS HANDBUCH DER PHARMAZEUTISCHEN PRAXIS", 1972, 4th edition, vol. 3, editors P. H. List and L. Hörhammer, pages 626-646, Springer-Verlag, Berlin, DE
- Antibiotika-Therapie in Klinik und Praxis, Simon et al, Schattauer Verlag, 1982, p. 9
- Antibiotika-Fibel, Otten et al, Georg Thieme Verlag Stuttgart, 1975, p. 202-205

## Description

### Background of the Invention

The present invention relates to an agent for preventing the infection with a methicillin resistant staphylococcus.

A staphylococcus, that is, Staphylococcus aureus, is a very common bacterium causing a bacterium-infectious disease, and is normally present in an affected part of purulent inflammation, in the environment and in the human skin, nose and pharynx. Since this bacterium produces exotoxins represented by an enterotoxin and exoenzymes, the bacterium has highly pathogenic properties and causes various diseases such as infectious diseases of the skin and flesh, infectious diseases of intestinal tracts (food poisoning), specticemia, endocarditis, cerebromeningitis, infectious diseases of the respiratory organs and infectious diseases of urinary passages. Furthermore, it is known that the bacterium causes staphylococcal scald skin diseases and so-called toxic shock diseases, that is, serious systemic diseases entailing various clinical symptoms such as sudden fever, eruption and hypotension. Since the staphylococcus is a bacterium, antibiotics are administered for prevention and remedy of these infectious diseases. However, the presence of a methicillin-resistant staphylococcus, which does not respond to the administration of antibiotics, has become known, and the remedy or prevention is greatly hindered.

Therefore, development of a medical agent capable of effectively preventing infectious diseases caused by a methicillin-resistant staphylococcus, which has no harmful adverse action to a human body and can be administered with safety, is eagerly desired.

### Summary of the Invention

Under this background, inventors searched for a substance capable of preventing the infection with a methicillin-resistant staphylococcus by killing the staphylococcus, from natural substances rather than chemical synthetic substances, and inventors found that a substance having such a function is present in tea and tea polyphenols. We have now completed the present invention based on this finding.

More specifically, in accordance with the present invention, there is provided an agent for preventing the infection with a methicillin-resistant staphylococcus, which comprises tea as an effective component.

### Brief Description of the Drawing

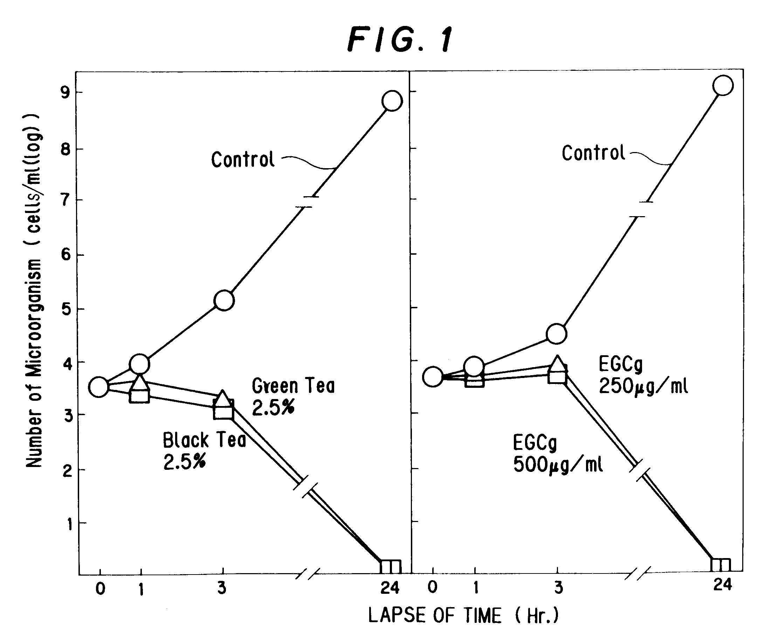

Fig. 1 is a graph illustrating the relation between the culture time and the number of cells.

### Detailed Description of the Invention

A principal component of tea is tea polyphenol compounds, and said tea polyphenol compounds include the tea catechin compounds represented by the general formula (I) given below and the theaflavin compounds represented by the general formula (II) given below, and also thearubigin: 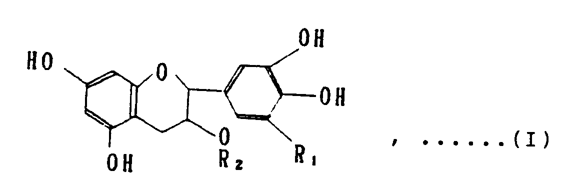
in which R₁ is a hydrogen atom or a hydroxy group and R₂ is a hydrogen atom or a 3,4,5-trihydroxy benzoyl group; and 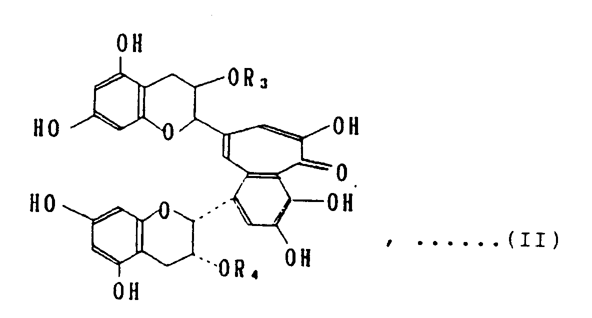
in which R₃ and R₄ are, each independently from the other, a hydrogen atom or a 3,4,5-trihydroxy benzoyl group.

Particular examples of the tea catechin compounds represented by the general formula (I) include: (-)epicatechin, which is a compound of the formula (I) with R₁ = H and R₂ = H; (-)epigallocatechin, which is a compound of the formula (I) with R₁ = OH and R₂ = H; (-)epicatechin gallate, which is a compound of the formula (I) with R₁ = H and R₂ = 3,4,5-trihydroxy benzoyl group; and (-)epigallocatechin gallate, which is a compound of the formula (I) with R₁ = OH and R₂ = 3,4,5-trihydroxy benzoyl group. Particular examples of the theaflavin compounds include: free theaflavin, which is a compound of the formula (II) with R₃ = H and R₄ = H; theaflavin monogallate A, which is a compound of the formula (II) with R₃ = 3,4,5-trihydroxy benzoyl group and R₄ = H; theaflavin monogallate B, which is a compound of the formula (II) with R₃ = H and R₄ = 3,4,5-trihydroxy benzoyl group; and theaflavin digallate, which is a compound of the formula (II) with R₃ = 3,4,5-trihydroxy benzoyl group and R₄ = 3,4,5-trihydroxy benzoyl group.

The above described tea polyphenol compounds can be prepared from tea leaves as the starting material and a method for the preparation thereof and a typical example of the product composition are described, for example, in Japanese Patent Kokai 59-219384, 60-13780 and 61-130285, etc.

When the agent for preventing the infection with the methicillin-resistant staphylococcus according to the present invention is used as a medical agent or is added to a food and the like, the above-mentioned tea polyphenol as the main component or tea is directly dissolved in water or an alcohol and the solution is used. Of course, an aqueous solution can be drunk or can be used as a hand-washing antiseptic solution. The concentration of tea at the time of application is preferably 1/10 to 1 of the ordinary drinking concentration (2 g/100 mℓ), and the concentration of the tea polyphenol is preferably 50 to 2000 ppm. It is especially preferred that the concentration of tea be 1/3 to 1 of the ordinary drinking concentration and the concentration of the tea polyphenol be 50 to 1000 ppm.

Since the agent for preventing the infection with a methicillin-resistant staphylococcus according to the present invention comprises a substance daily drunk in a considerable quantity as the main component, there is no fear of adverse side effects on a human body and this agent shows a strong antibacterial activity on methicillin-resistance staphylococcus. Therefore, the agent for preventing the infection with a methicillin-resistant staphylococcus according to the present invention is very effective for preventing the infection with a methicillin-resistant staphylococcus.

### Example 1

### (1) Preparation of Tea Extract, Tea Polyphenols and Solution of Antibiotic

Black tea and green tea extracts were prepared by carrying out extraction with a phosphate buffer saline (PBS) so that the concentration was 20 w/v%. Furthermore, (-)-epigallocatechin gallate (EGCg), theaflavin digallate (TF3) and minomycin (MINO) as a control antibiotic were independently dissolved in PBS at concentrations of 1.25 mg/mℓ, 2.5 mg/mℓ and 5 µg/mℓ, respectively.

### (2) Strains

As MRSA, there were used 30 strains separated at the Showa University Hospital, 22 strains separated at the Fujigaoka Hospital of Showa University and one other strain, 53 strains as a whole. As the standard strain, there were used Staphylococcus aureus ATCC 25923, Staphylococcus aureus ATCC 12598, Staphylococcus aureus ATCC 209, and three other strains, 6 strains as a whole. Furthermore, 20 strains of Staphylococcus aureus separated from foodpoisoning materials were used at the experiments.

### (3) Measurement of Antibacterial Activity

The measurement of the antibacterial activities of black tea and green tea extracts, EGCg, TF3, and MINO as the control was carried out according to the customary agar well (sample: 50 µℓ) method using Mueller-Hinton medium (10 mℓ). Moreover, the test was conducted with antibiotic substances, ampicillin (PcA, 2 µg, 5 µg, 20 µg) and oxacillin (PcM, 0.5 µg, 2 µg, 10 µg) according to the three-concentration disk method.

As a result, it was found that among 53 strains of MRSA, 25 strains were resistant to MINO. All of the 53 strains were resistant to PcM, and 28 strains were resistant to PcA and 25 strains were slightly resistant to PcA. In contrast, all of the strains were sensitive to black tea, green tea, EGCg and TF3, and it was found that black tea, green tea, EGCg and TF3 had an effective antibacterial activity. The standard strains and the strains of Staphylococcus aureus derived from the food poisoning materials were sensitive to MINO, PcA and PcM and also sensitive to green tea, black tea, EGCg and TF3.

### Example 2

It was checked whether or not the propagation in a culture medium of MRSA could be inhibited. Culture media were prepared by adding black tea (2.5%), green tea (2.5%) or EGCg (250 or 500µg/mℓ) to ordinary bouillon, and an additive-free control bouillon medium was similarly prepared. Each medium was inoculated with an equal number of cells of the Hatanodai strain (separated from a patient) of MRSA, and culturing was carried out according to customary procedures.

The relation between the culture time and the number of cells is shown in Fig. 1. It is seen from Fig. 1 that in the control medium, the number of cells logarithmically increased, and that when green tea, black tea or EGCg was added, the number of cells gradually decreased and cells were completely annihilated after 24 hours.

## Claims

1. Use of tea for the preparation of an agent for preventing the infection with a methicillin-resistant Staphylococcus aureus.

2. The use as claimed in claim 1, wherein a tea polyphenol which is an ingredient of tea is used for the preparation of the agent.

3. The use as claimed in claim 2, wherein the tea polyphenol is at least one member selected from the group consisting of epigallocatechin gallate, epicatechin gallate, epigallocatechin, epicatechin, (+)-catechin, isomers thereof, free theaflavin, theaflavin monogallate A, theaflavin monogallate B and theaflavin digallate.

## Patentansprüche

1. Verwendung von Tee für die Herstellung eines Mittels zur Verhütung von Infektionen mit einem Methicillin-resistenten Staphylococcus aureus.

2. Die Verwendung wie in Anspruch 1 beansprucht, wobei ein Tee-Polyphenol, das ein Bestandteil von Tee ist, für die Herstellung des Mittels verwendet wird.

3. Die Verwendung wie in Anspruch 2 beansprucht, wobei das Tee-Polyphenol mindestens ein Bestandteil ist, ausgewählt aus der aus Epigallokatechingallat, Epikatechingallat, Epigallokatechin, Epikatechin, (+)-Katechin, Isomeren davon, freiem Theaflavin, Theaflavinmonogallat A, Theaflavinmonogallat B und Theaflavindigallat bestehenden Gruppe.

## Revendications

1. Utilisation de thé dans la préparation d'un agent destiné à la prévention d'une infection due à un Staphylococcus aureus résistant à la méthicilline.

2. Utilisation selon la revendication 1, caractérisée en ce qu'un composant polyphénolique du thé est utilisé dans la préparation dudit agent.

3. Utilisation selon la revendication 2, caractérisée en ce que le composant polyphénolique du thé est choisi parmi au moins l'un des membres du groupe constitué par le gallate d'epigallocatéchine, le gallate d'epicatéchine, l'epigallocatéchine, l'epicatéchine, la (+)-catéchine, leurs isomères, la théaflavine à l'état libre, le monogallate A de théaflavine, le monogallate B de théaflavine et le digallate de théaflavine.
